# EUROPEAN PATENT APPLICATION

(11) **EP 3 053 514 A1**
(43) Date of publication of application: **10.08.2016**
(21) Application number: 14850255.2
(22) Date of filing: 27.08.2014
(51) Int. Cl.: A61B 5/00, G06T 1/00

(54) **ORGAN IMAGING APPARATUS**

(30) Priority: 01.10.2013 JP 2013206623
(71) Applicant: Konica Minolta, Inc., Tokyo 100-7015 (JP)
(72) Inventor: MATSUDA, Shinya, Tokyo 100-7015 (JP)
(74) Representative: Gille Hrabal
(86) International application number: PCT/JP2014/072408
(87) International publication number: WO 2015/049936

(57) **Abstract**

The organ imaging apparatus (1) is provided with an image pickup unit (3) for acquiring images by imaging an organ in a living body and a detection unit (6) for detecting a feature of the organ on the basis of the images acquired by the image pickup unit (3). The detection unit (6) extracts blue component image data from the images taken of the organ and detects the shininess of the organ surface on the basis of the extracted blue component image data alone.

## Description

### Technical Field

The present invention relates to an organ imaging device that detects, based on an image obtained by imaging an organ of a living body, the degree of gloss on the surface of the organ.

### Background Art

In Oriental medicine, a method of diagnosis (tongue diagnosis) is known in which the condition of the human tongue is inspected to diagnose health condition and disease condition. In tongue diagnosis, an examinee's physical condition and healthiness are examined based on the color and shape of the tongue (more precisely, the tongue and the coating on it).

One item of inspection in tongue diagnosis is the moisture (moistness) on the surface of the tongue. When the body is short of water, reduced moisture in the tongue cells makes the surface of the tongue dry. This condition, categorized as body fluid shortage in Oriental medicine, results from fever and inflammation. When aggravated, the condition may lead to a serious condition such as dehydration.

Such diagnosis is practiced by specialized physicians, who, however, rely on experience and intuition. Thus, diagnoses tend to vary from one practitioner to another and be far from objective. Moreover, obscure memories of past condition hamper an objective grasp of changes in condition.

As a solution, there have been proposed systems in which a subject is imaged with a digital camera and, from the taken image, features are quantified and recorded to make a diagnosis. For example, according to Patent Document 1, illumination light is applied to the tongue, and the reflected light is received by a camera. From an image thus taken, color-clipped pixels, that is, pixels that are white and have particular luminance (90% or more of the maximum luminance value), are detected, and by counting the number of those pixels, the moisture on the tongue is measured. That is, based on luminance information included in the light reflected from the tongue, the degree of gloss on the surface of the tongue is detected.

On the other hand, according to Patent Document 2, there are provided an integrating sphere that has a plurality of openings formed in it; a diffusion light source device that shines light on the integrating sphere such that an examinee is not directly irradiated with the light through any of the openings of the integrating sphere; and a gloss light source that shines light such that the light specularly reflected from the tongue surface enters a camera. By alternating between imaging of the tongue with the gloss light source extinguished and imaging of the tongue with the gloss light source lit, image data including only color but not gloss on the tongue surface and image data including both color and gloss on the tongue surface are acquired. Through differential calculation subtracting the former image data from the latter image data, image data including only gloss is acquired.

### List of Citations

### Patent Literature

Patent Document 1: JP-2005-137756 (see paragraphs [0082]-[0084], Fig. 9, etc.)
Patent Document 2: JP-2011-239926 (see paragraphs [0024]-[0025], Figs. 1, 2, etc.)

### Summary of the Invention

### Technical Problem

For each pixel in a taken image, let the red, green, and blue image data be represented by R, G, and B respectively, and let the luminance data be represented by Y, then the luminance data Y of the pixel is generally given by the following formula.

Y=0.22R+0.71G+0.07B

Thus, the luminance data Y includes R, G, and B color information. Accordingly, as any of R, G, and B varies, Y too varies. In particular, in a case where the target organ is the tongue, the R and G color components in a taken image of the tongue tend to vary more with the health condition of the examinee and differences among individuals than the B color component. Thus, with a method where, as in Patent Document 1, the degree of gloss on the surface of the tongue is detected based on luminance information including at least R and G color information, as the redness of the tongue (the R component) and the whiteness of the coating on it (chiefly the G component) vary with the health condition of the examinee and differences among individuals, the luminance information varies, letting the detected degree of gloss vary. For example, in a case where the tongue is pale red and the coating is white and thick, higher luminance yields a higher degree of gloss. This degrades the accuracy of degree-of-gloss detection.

On the other hand, in Patent Document 2, to obtain image data including only gloss on the tongue surface, as mentioned above, image data including only color on the tongue surface and image data including both color and gloss on the tongue surface are acquired and are subjected to differential calculation. Here, obtaining image data including only color requires the use of an integrating sphere for sufficiently diffusing illumination light. This requires large equipment and incurs increased cost.

Devised to provide a solution to the above-discussed problems, the present invention aims to provide an organ imaging device that can detect the degree of gloss on the surface of an organ accurately with a compact, low-cost configuration.

### Means for Solving the Problem

According to one aspect of the present invention, an organ imaging device includes: an imager for imaging an organ of a living body to acquire an image; and a detector for detecting a feature of the organ based on the image acquired by the imager. The detector extracts the image data of a blue component from the taken image of the organ, and detects the degree of gloss on the surface of the organ based only on the extracted image data of the blue component.

### Advantageous Effects of the Invention

With the above configuration, it is possible to detect the degree of gloss on the surface of an organ accurately with a compact, low-cost configuration.

### Brief Description of Drawings

[Fig. 1] is a perspective view showing an exterior appearance of an organ imaging device according to one embodiment of the present invention;
[Fig. 2] is a block diagram showing an outline of a configuration of the organ imaging device;
[Fig. 3] is a diagram illustrating the illumination angle of an imaging object with respect to the organ imaging device;
[Fig. 4] is a diagram illustrating a taken image of the tongue, an edge extraction filter, and a contour line of the organ extracted by use of the edge extraction filter;
[Fig. 5] is a diagram illustrating a taken image of the tongue along with a sectional shape of the tongue;
[Fig. 6] is a diagram illustrating a positional relationship between a contour line of the tongue extracted from the taken image and degree-of-gloss detection areas;
[Fig. 7] is a graph showing a spectrum distribution on the tongue;
[Fig. 8] is a graph showing a frequency distribution of B image data extracted from the detection areas;
[Fig. 9] is a diagram illustrating a relationship between the number of high-value pixels and diagnoses by a *Kampo* doctor; and
[Fig. 10] is a flow chart showing a flow of operation in the organ imaging device.

### Description of Embodiments

Hereinafter, an embodiment of the present invention will be described with reference to the accompanying drawings. In the present description, a range of values from A to B is assumed to include the lower limit A and the upper limit B.

### [Overcall Configuration of an Organ Imaging Device]

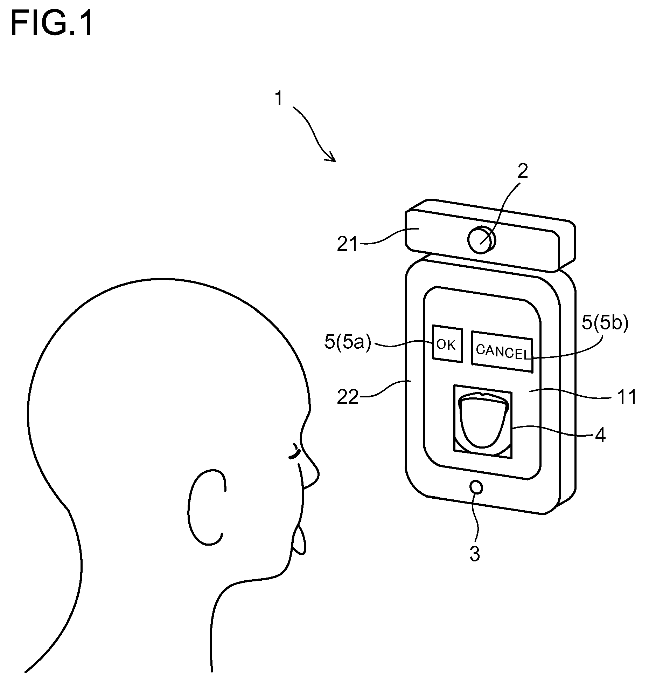

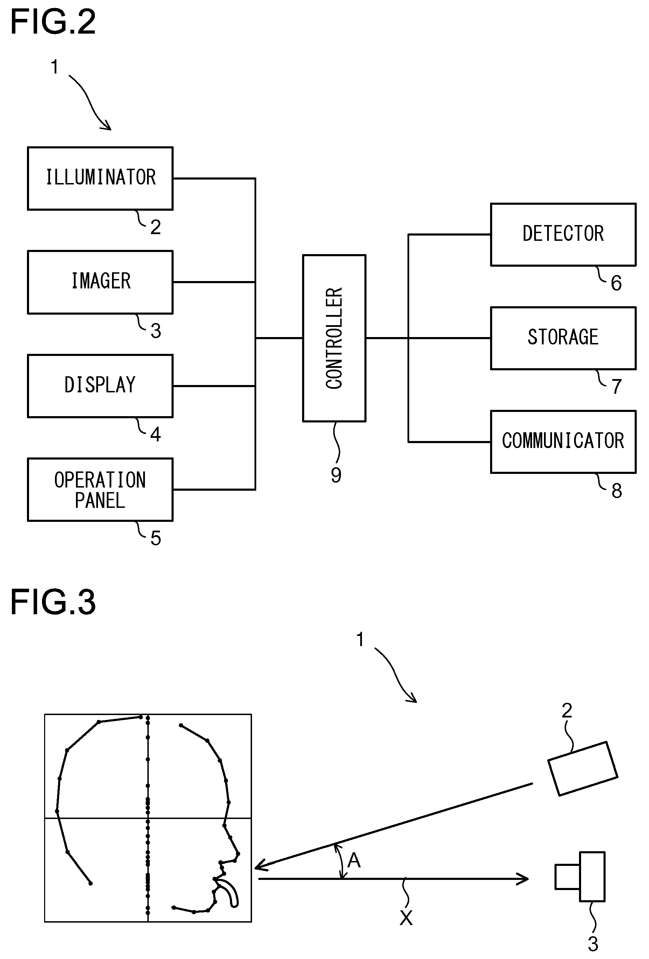

Fig. 1 is a perspective view showing an exterior appearance of an organ imaging device 1 according to one embodiment of the present invention, and Fig. 2 is a block diagram showing an outline of a configuration of the organ imaging device 1. The organ imaging device 1 is used to image an organ of a living body to extract information needed for diagnosis. The following description deals with an example where the imaging object is the tongue as an organ of a living body and the information needed for diagnosis is the degree of gloss (the degree of moistness) on the surface of the tongue.

The organ imaging device 1 includes an illuminator 2, an imager 3, a display 4, an operation panel 5, a detector 6, a storage 7, a communicator 8, and a controller 9. The illuminator 2 is provided in a housing 21, and the blocks other than the illuminator 2 (e.g., the imager 3, the display 4, and the operation panel 5) are provided in a housing 22. The housings 21 and 22 are coupled together so as to be rotatable relative to each other. The illuminator 2 and the other blocks may be provided in a single housing. The organ imaging device 1 may be configured as a multifunction portable information terminal.

The illuminator 2 is configured as a light that illuminates an imaging object from above. The illuminator 2 includes a light source that emits light of a daylight color, such as a xenon lamp, for improved color rendering. The brightness of the light source varies depending on the sensitivity of the imager 3 and the distance to the imaging object; the brightness can be, for example, such as to provide an illumination of 1000 to 10000 lx at the imaging object. The illuminator 2 includes, in addition to the light source, a lighting circuit and a dimming circuit, and is controlled according to instructions from the controller 9 so as to be lit, extinguished, and dimmed.

The imager 3 images an organ of a living body to acquire an image of it under the illumination of the illuminator 2, and includes an imaging lens and an area sensor (image sensor). The aperture of the imaging lens (the fastness of the lens), the shutter speed, and the focal length are so set that the imaging object is in focus over its entire area. For example, the f-number can be set at 16, the shutter speed can be set at 1/120 seconds, and the focal length can be set at 20 mm.

The area sensor comprises, for example, an image sensor such as a CCD (charge-coupled device) image sensor or a CMOS (complementary metal oxide semiconductor) sensor, and its sensitivity, resolution, etc. are so set that the color and shape of the imaging object can be detected satisfactorily. For example, the sensitivity can be set at 60 db, and the resolution can be set at 10 megapixels.

The imaging by the imager 3 is controlled by the controller 9. The imager 3 further includes, in addition to the imaging lens and the area sensor, a focusing mechanism, an aperture mechanism, a drive circuit, an A/D conversion circuit, etc., of which none is illustrated, and is controlled according to instructions from the controller 9 in terms of focus, aperture, A/D conversion, etc. The imager 3 acquires, as the data of a taken image, data comprising, for example, eight bits, representing a value from 0 to 255, for each of red (R), green (G), and blue (B) per pixel.

The display 4 includes a liquid crystal panel, a backlight, a lighting circuit, and a control circuit, of which none is illustrated, and displays the image acquired by the imaging by the imager 3 according to instructions from the controller 9. The display 4 can also display information (e.g., the result of a diagnosis made by an external medical facility based on information transmitted to it) acquired from outside via the communicator 8.

The operation panel 5 comprises an input device from which to instruct the imager 3 to perform imaging, and includes an OK button (TAKE IMAGE button) 5a and a CANCEL button 5b. In the embodiment, the display 4 and the operation panel 5 are constituted by a single touch panel display device 11, and separate display areas are provided on the touch panel display device 11, one for the display 4 and the other for the operation panel 5. The operation panel 5 may be configured as any other input device than the touch panel display device 11 (the operation panel 5 may be provided anywhere else than inside the display area of the touch panel display device 11).

The detector 6 includes an unillustrated processor, and detects a feature of an organ based on an image acquired by the imager 3. In particular, the detector 6 extracts, from a taken image of an organ, a contour line of the organ; then sets an area for the detection of the degree of gloss with reference to the extracted contour line; then extracts, from pixels in the detection area, the B image data out of red (R), green (G), and blue (B) components; then detects the degree of gloss on the surface of the organ based on the extracted B image data. How a contour line is extracted, how a detection area is set, and how the degree of gloss is detected will be described in detail later.

The storage 7 comprises a memory that stores the data of images acquired by the imager 3, information acquired by the detector 6, information received from outside, etc. The communicator 8 comprises an interface for transmitting image data and information as mentioned above to outside via a communication network (which may be wired or wireless), and for receiving information from outside. The controller 9 controls the operation of relevant blocks in the organ imaging device 1, and comprises, for example, a CPU (central processing unit) and a memory, with programs for controlling different blocks stored in the latter.

### [Examples of Arrangement of the Illuminator and the Imager]

Fig. 3 is a diagram illustrating the illumination angle of an imaging object with respect to the organ imaging device 1. As shown there, the imager 3 is arranged right in front of the imaging object (the tongue or the face). The illuminator 2 is so arranged as to illuminate the imaging object, for example, at an angle A of 0° to 45° relative to the imaging optical axis X of the imager 3, which passes through the imaging object. The imaging optical axis X denotes the optical axis of the imaging lens provided in the imager 3.

Here, when illumination is applied at a large angle A, the shadow of the upper lip reduces the area over which the tongue can be imaged. Conversely, when illumination is applied at a small angle A, specular reflection causes severe color clipping. Out of these considerations, a preferred range of the angle A for illumination is from 15° to 30°.

### [Extracting a Contour Line of an Organ]

In the embodiment, the detector 6 extracts a luminance edge in the taken image (a part of the image where luminance changes sharply), and thereby extracts a contour line of the tongue as an organ.

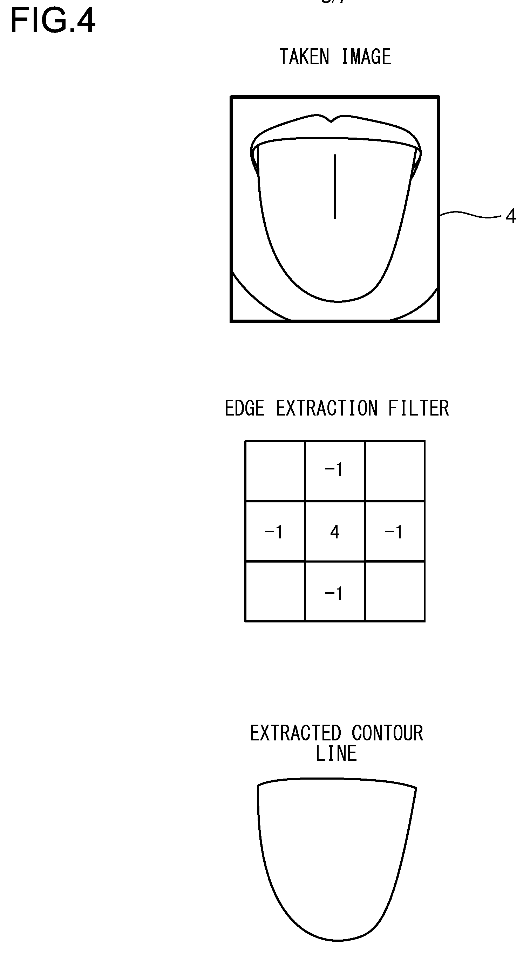

A luminance edge can be extracted, for example, by use of an edge extraction filter as shown in Fig. 4. An edge extraction filter is a filter that gives weights to pixels near a pixel of interest when performing first-order differentiation (when calculating differences in image data between neighboring pixels). By use of such an edge extraction filter, for example, with respect to the G image data of each pixel in the taken image, differences in image data are calculated between the pixel of interest and neighboring pixels, and those pixels which yield differences exceeding a predetermined threshold value are extracted; in this way, pixels that constitute a luminance edge can be extracted. Around the tongue, its shadow produces brightness differences; thus, by extracting pixels that constitute a luminance edge in the manner described above, it is possible to extract a contour line of the tongue. Here, G image data is used in the calculation because it has the greatest influence on luminance; R or B image data may be used instead.

In Oriental medicine, a white patch of coating seen in a central part of the tongue is called "tongue coating", and its color is called "tongue coating-color". On the other hand, the color of the rest, the red part, of the tongue is called "tongue-color".

### [Setting a Detection Area]

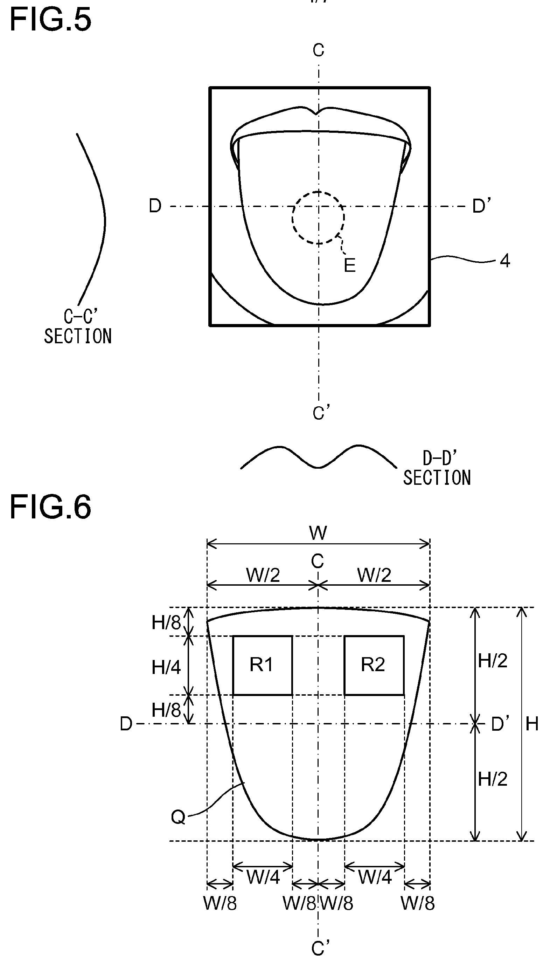

Fig. 5 shows a taken image of the tongue along with a sectional shape of the tongue. When the tongue is imaged, it is protracted out of the oral cavity. To permit the imager 3 to image the upper lip-side surface of the protracted tongue, this surface is curved so as to be convex toward the imager 3 (see the C-C' section). As necessary, how to protract the tongue can be explained in a specification or instruction manual of the device so that the examinee can hold the tongue in a proper imaging position.

When the tongue is imaged with the illuminator 2 and the imager 3 arranged as shown in Fig. 3, a specular reflection area appears in the upper half of the tongue (because the illuminator 2 is located above the imaging optical axis X). On the other hand, in the left/right direction with respect to the tongue, a middle part of the tongue and the left and right ends of the tongue sag to describe an M-shape (see the D-D' section). The tongue has a largely similar sectional shape from an upper to a lower part of it. Moreover, in a central part of the tongue, a pattern of cracks may be observed. Accordingly, in the embodiment, illumination is applied at an angle of 15 degrees, and an area on the upper half of the tongue excluding a middle part and opposite end parts of it in the left/right direction is set as an area suitable for the detection of the degree of gloss.

More specifically, as shown in Fig. 6, from a contour line Q of the tongue extracted from a taken image through the process described above, the detector 6 detects the top, bottom, left, and right ends of the tongue to determine the top-to-bottom length H and the left-to-right width W of the tongue. Then, with reference to the contour line Q of the tongue, the detector 6 sets degree-of-gloss detection areas R1 and R2 such that they have a positional relationship as shown in Fig. 6.

The degree-of-gloss detection areas R1 and R2 may be set without extracting a contour line Q of the tongue as described above. For example, as by displaying frame lines on the display 4 to guide the tongue into a proper imaging position, it is possible to image the tongue in the proper position for each individual living body; it is then possible to set predetermined areas in a taken image of the tongue as the degree-of-gloss detection areas R1 and R2. This eliminates the need to extract a contour line Q, and thus helps reduce the time required for degree-of-gloss detection, simplify the circuit configuration of the detector 6, and so forth.

### [How to Detect the Degree of Gloss]

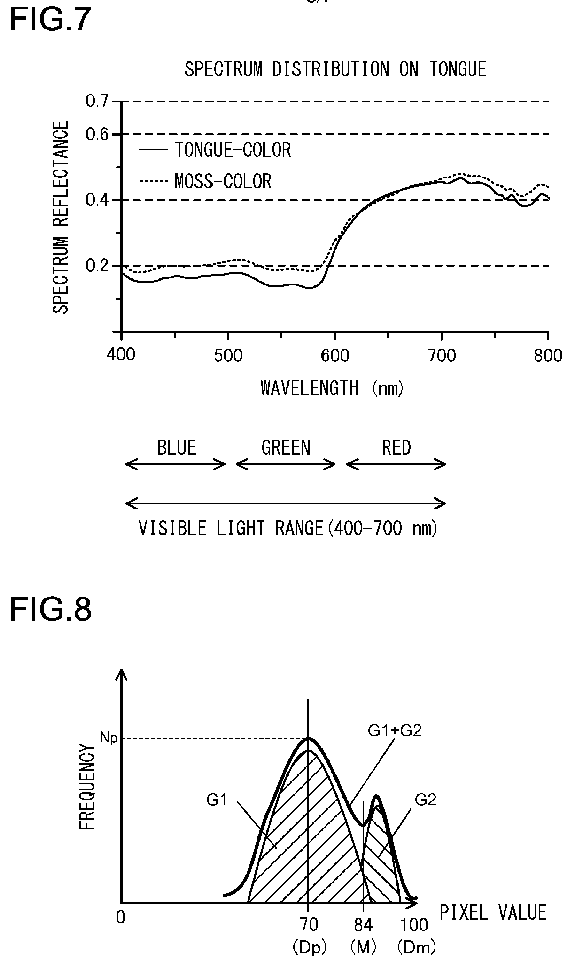

Fig. 7 is a graph showing a spectrum distribution on the tongue. The tongue is a mucous membrane without epidermis, and its color reveals the color of blood. In the color of blood, the proportion of the R component (with wavelengths from 600 nm to 700 nm) is high, and the proportion of the B component (with wavelengths of 500 nm or less) is low. A lighter tongue color yields a lower proportion of the R component, and a darker tongue color yields a higher proportion of the R component.

On the other hand, the tongue coating is formed of cornified cells of papillae, and takes on a white to yellow color. With a thin tongue coating, the color of the tongue beneath prevails, and thus the proportion of the R component is high, as shown in Fig. 7. With a white, thick tongue coating, the proportion of the G component (with wavelengths from 500 nm to 600 nm) is high.

While the colors of the tongue and the tongue coating vary as described above with the health condition of the living body and differences among individuals, the proportion of the B component varies little. Accordingly, in the embodiment, the degree of gloss on the surface of the tongue is detected based on the B image data obtained from a taken image of the tongue through a procedure as described below.

First, the detector 6 extracts B image data from pixels in the degree-of-gloss detection areas R1 and R2 of the taken image, and creates a frequency distribution of the data. Fig. 8 schematically shows a frequency distribution of the extracted B image data. In Fig. 8, the horizontal axis represents the B pixel value (image data), and the vertical axis represents the frequency (the number of pixels). Here, however, for simplicity's sake, it is assumed that a pixel value takes a value from 1 to 100, a greater pixel value indicating higher luminance.

Next, from the above-mentioned frequency distribution, the detector 6 determines the pixel value Dp that corresponds to the highest frequency Np (in the example in Fig. 8, Dp = 70). The detector 6 then multiplies that pixel value Dp by 1.2 to determine the threshold value M (in the example in Fig. 8, M = 84). Then, across the range from the threshold value M to the maximum value of the image data (the maximum pixel value Dm = 100), the detector 6 cumulates (adds up) the frequencies to determine the number of high-value pixels. The pixel value Dp may instead be determined by first finding a function that continuously represents how the frequency varies, then smoothing and removing noise from the function, and then determining the pixel value Dp corresponding to the highest frequency Np. The number of high-value pixels may be determined by integrating the smoothed function across a predetermined range.

Here, if no specular reflection occurs on the tongue surface during imaging, the frequency distribution of the B image data is composed of a single distribution close to a normal distribution (a first group G1). By contrast, if specular reflection occurs, the first group G1 is accompanied by another distribution (a second group G2) with high frequencies at higher pixel values. In addition, since the B image data varies little with the health condition of the living body and differences among individuals as mentioned above, the width of the first group G1 (the width from the minimum to the maximum pixel value in the first group G1) is smaller than the frequency distribution (normal distribution) of other image data, such as of R or G. As a result, a clear border between the first and the second groups G1 and G2 (a point at which the frequency has a minimum, turning from decrease to increase) appears between the value of image data at which the frequency is highest (i.e., the pixel value Dp) and the maximum value of image data (i.e., the maximum pixel value Dm). Thus, the first and the second groups G1 and G2 can be distinguished from each other easily. In detecting the degree of gloss, it is preferable to do so not based on the first group G1, which includes no gloss component (specular reflection component), but based on the second group G2, which represents the gloss component.

Accordingly, the detector 6 sets the threshold value M greater than the pixel value Dp, and determines the sum of frequencies between the threshold value M and the maximum pixel value Dm as the number of high-value pixels, so as to obtain a value close to the sum of frequencies in the second group G2.

In particular, it has been experimentally found out that, in a frequency distribution of B image data, the boundary between the first and the second groups G1 and G2 appears in the range from 1.1 to 1.3 times the pixel value Dp. Accordingly, in the embodiment, the detector 6 sets the threshold value M within the range from 1.1 to 1.3 times the pixel value Dp (in the example in Fig. 8, 1.2Dp = 84), and determines the sum of frequencies between the threshold value M and the maximum pixel value Dm as the number of high-value pixels.

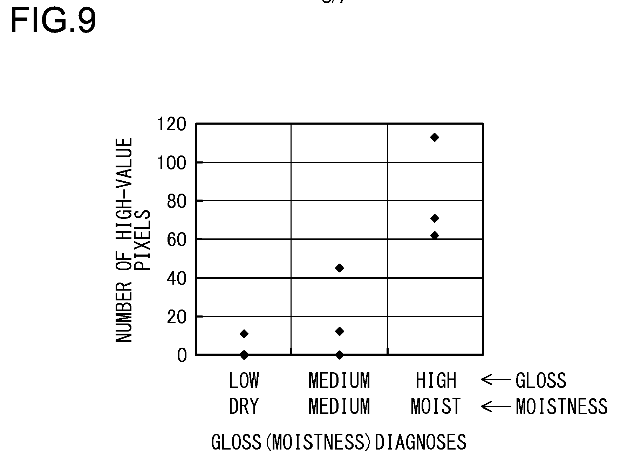

With each of a plurality of examinees, the tongue was imaged as a sample, a frequency distributions of the B image data was created, and from this frequency distribution, the number of high-value pixels was determined in the manner described above. Fig. 9 shows a correlation between the thus determined numbers of high-value pixels and the results of actual diagnosis of the moistness of the tongue in those examinees by a *Kampo* doctor (*Kampo* is a Japanese version of ancient Chinese medicine). The diagram reveals a high correlation between the number of high-value pixels and diagnoses made by a *Kampo* doctor. Specifically, it can be said that the fewer the high-value pixels, the drier the tongue (the lower the degree of gloss), and that the more the high-value pixels, the more moist the tongue (the higher the degree of gloss). Thus, with the correlation in Fig. 9 stored in the form of a table in the storage 7, the detector 6 can refer to it to detect, based on the detected number of high-value pixels, the degree of gloss and the degree of moistness on the surface of the tongue.

### [Control Flow]

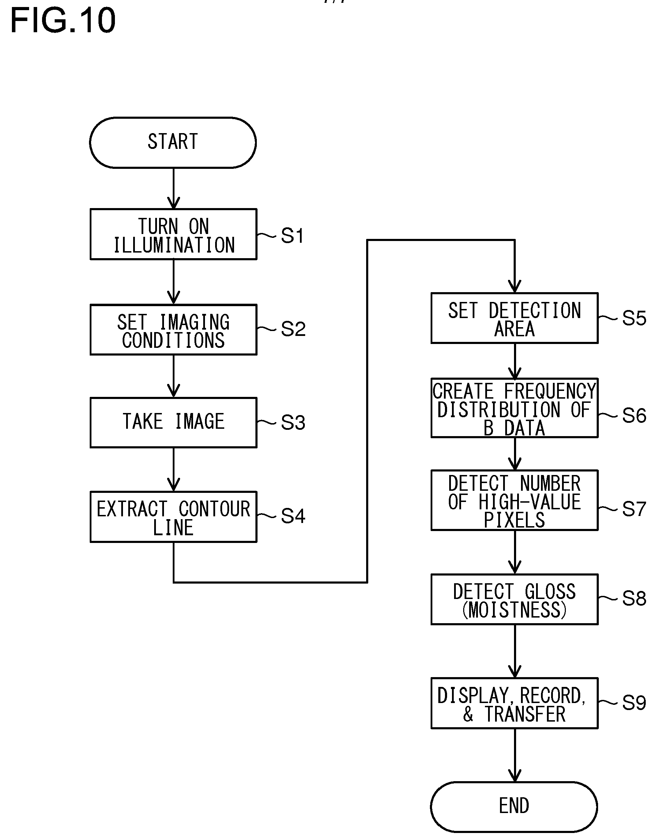

Fig. 10 is a flow chart showing the flow of operation in the organ imaging device 1 according to the embodiment. In the organ imaging device 1, in response to an instruction to image from the operation panel 5 or from an unillustrated input device, the controller 9 lights the illuminator 2 (S1), and sets imaging conditions (S2). On completion of the setting of imaging conditions, the controller 9 controls the imager 3 so as to image the tongue as an imaging object (S3).

On completion of the imaging, the detector 6 extracts a contour line Q of the tongue from the taken image of the tongue (S4). Then, the detector 6 detects the top, bottom, left, and right ends of the tongue from the extracted contour line Q, and sets degree-of-gloss detection areas R1 and R2 with reference to the contour line Q (S5). Subsequently, the detector 6 extracts the B image data from pixels in the set degree-of-gloss detection areas R1 and R2 to create a frequency distribution (S6), and calculates the number of high-value pixels. The detector 6 then refers to the table containing the relationship between the number of high-value pixels and diagnoses, detects and quantifies the degree of gloss (the degree of moistness) on the tongue based on the calculated number of high-value pixels, and, based on the results, diagnoses the healthiness of the examinee (S8). The result of the detection of the degree of gloss and the result of the diagnosis of the examinee's healthiness are displayed on the display 4, and are, as necessary, output (recorded) to an unillustrated output device, or transferred to outside via the communicator 8 (S9). The result of degree-of-gloss detection may be quantified and transmitted to outside so that the examinee's health condition is diagnosed outside.

As described above, the detector 6 extracts, from a taken image of an organ, B image data, which varies less than R or B image data with the health condition of the living body or differences among individuals; it then detects, based on the extracted B image data (based only on B image data, not B or R image data), the degree of gloss on the surface of the organ. In this way, compared with a configuration where the degree of gloss is detected based on luminance information containing R and G color information, it is possible to reduce the influence of the health condition of the living body or differences among individuals (the influence of variations in the R and G components), and thus to detect the degree of gloss more accurately. In addition, with a configuration where the degree of gloss is directly detected based on the B image data extracted from a taken image, it is possible to detect the degree of gloss without the use of a large integrating sphere as conventionally used, and thus to detect the degree of gloss on the surface of an organ accurately with a compact, low-cost configuration.

In particular, in a case where the target organ is the tongue, by detecting the degree of gloss in the manner described above, the detector 6 can detect the degree of moistness on the tongue accurately.

Moreover, the detector 6 creates a frequency distribution of the extracted B image data, and detects the degree of gloss based on the sum of frequencies (the number of high-value pixels) between a threshold value M greater than the value of image data at which the frequency is highest (the pixel value Dp) and the maximum value of image data (the maximum pixel value Dm). Thus, it is possible to obtain a detection result close to that based on the sum of frequencies in a distribution (the second group G2) representing the gloss component in the frequency distribution of B image data, and thus to further enhance the accuracy of degree-of-gloss detection.

Moreover, the threshold value M equals 1.1 to 1.3 times the value of image data at which the frequency is highest. Thus, it is possible to make the result of degree-of-gloss detection based on the number of high-value pixels as close as possible to that based on the sum of frequencies in the second group G2 representing the gloss component, and thus to reliably enhance the accuracy of degree-of-gloss detection.

Moreover, the detector 6 sets degree-of-gloss detection areas R1 and R2 with reference to a contour line Q of an organ. Thus, even when the contour line Q of the organ varies among different living bodies (among individuals), it is possible to set areas suitable for degree-of-gloss detection for each living body. Then, it is possible, based on the B image data extracted from pixels in the degree-of-gloss detection areas R1 and R2, to detect the degree of gloss on the surface of the organ properly.

### [Other]

Although the above description deals with a case where the imaging object is the human tongue, the living body (anything alive) does not necessarily have to be a human but may be any animal other than a human. For example, also with the tongue of a pet or other domestic animal, the procedure according to the embodiment can be used to detect the degree of gloss on the surface of the tongue, and to make a diagnosis based on the detection result. In that way, it is possible to recognize poor health condition of an animal, which cannot communicate by words.

Moreover, the organ of a living body that can be taken as an imaging object is not limited to the tongue. For example, it can instead be the lips, or a part inside the oral cavity, such as the gum, or the lining of the stomach or the intestines, or the underside of the eyelid. It is possible to detect the degree of gloss on the surface of an organ simultaneously when checks are made in respective medical specialties.

Put another way, the organ imaging device described above is configured as noted below, and provides benefits as noted below.

An organ imaging device includes: an imager for imaging an organ of a living body to acquire an image; and a detector for detecting a feature of the organ based on the image acquired by the imager. The detector extracts the image data of a blue component from the taken image of the organ, and detects the degree of gloss on the surface of the organ based only on the extracted image data of the blue component.

The image data of the blue component (B) included in the taken image of the organ varies less with the health condition of the living body or differences among individuals than the image data of, for example, a red component (R) or a green component (G). Thus, with a configuration where the detector extracts the B image data, which varies little, from the taken image of the organ and detects the degree of gloss on the surface of the organ based on the extracted B image data, compared with a configuration where the degree of gloss is detected based on luminance information including color information of R and G, it is possible to reduce the influence of the health condition of the living body or differences among individuals, and thus to detect the degree of gloss accurately. In addition, with a configuration where the degree of gloss is detected directly based on the B image data extracted from the taken image, it is not necessary to use a large integrating sphere for acquiring only color data as conventionally used in a configuration where data including both color and gloss and data including only color are acquired and the degree of gloss is detected through differential calculation. Thus, it is possible to detect the degree of gloss on the surface of the organ accurately with a compact. low-cost configuration.

The organ can be a tongue, and the detector can detect the degree of moistness on the tongue by detecting the degree of gloss. In this way, it is possible to detect the degree of moistness on the tongue accurately.

The detector can detect the degree of moistness by referring to a table containing a correlation between the blue component data and diagnoses by a *Kampo* doctor. In this way, it is possible to detect the degree of moistness on the tongue accurately according to diagnoses by a *Kampo* doctor.

The detector can create a frequency distribution of the extracted image data of the blue component, and detects the degree of gloss based on the sum of frequencies between a threshold value greater than the value of image data corresponding the highest frequency and the maximum value of image data.

If no specular reflection occurs on the surface of the organ during imaging, the frequency distribution of the extracted B image data is a distribution (a first group) close to a normal distribution; if specular reflection occurs, the above distribution observed without specular reflection is accompanied by another distribution (a second group) with high frequencies at higher pixel values (image data). By detecting the degree of gloss based on the sum of frequencies between a threshold value greater than the value of image data corresponding to the highest frequency and the maximum value of image data, it is possible to obtain a detection result close to that based on the sum of frequencies in the second group, and thus to further enhance the accuracy of degree-of-gloss detection.

It is preferable that the threshold value equal 1.1 to 1.3 times the value of image data corresponding to the highest frequency.

Since the B image data varies little with the health condition of the living body or differences among individuals, the width of the above-mentioned first group is smaller than the width of the frequency distribution of other image data, such as of R or G. It has been found out that the border between the first and second group lies within the range of 1.1 to 1.3 times the value of image data corresponding to the highest frequency. Accordingly, by setting the threshold value equal to 1.1 to 1.3 times the value of image data corresponding the highest frequency, it is possible to make the result of degree-of-gloss detection based on the sum of frequencies between the threshold value and the maximum value of image data as close as possible to that based on the sum of frequencies in the second group, and thus to reliably enhance the accuracy of degree-of-gloss detection.

The detector can extract a contour line of the organ from the taken image of the organ and then set a detection area for detection of the degree of gloss with reference to the extracted contour line, and the image data of the blue component can be extracted from pixels in the detection area.

With a configuration where the detector sets a detection area for detection of the degree of gloss with reference to a contour line of the organ, even when the contour line of the organ varies among different living bodies, it is possible to set an area suitable for degree-of-gloss detection for each living body. Then, it is possible, based on the B image data extracted from pixels in the detection area, to detect the degree of gloss on the surface of the organ properly.

### Industrial Applicability

The present invention finds applications in devices that detect, from an image obtained by imaging an organ of a living body, the degree of gloss on the surface of the organ.

### List of Reference Signs

- 1: organ imaging device

- 3: imager
- 6: detector

## Claims

1. An organ imaging device, comprising:
an imager for imaging an organ of a living body to acquire an image; and
a detector for detecting a feature of the organ based on the image acquired by the imager, wherein
the detector extracts image data of a blue component from the taken image of the organ, and detects a degree of gloss on a surface of the organ based only on the extracted image data of the blue component.

2. The organ imaging device of claim 1, wherein
the organ is a tongue, and
the detector detects a degree of moistness on the tongue by detecting the degree of gloss.

3. The organ imaging device of claim 2, wherein
the detector detects the degree of moistness by referring to a table containing a correlation between the blue component data and diagnoses by a *Kampo* doctor.

4. The organ imaging device of any one of claims 1 to 3, wherein
the detector creates a frequency distribution of the extracted image data of the blue component, and detects the degree of gloss based on a sum of frequencies between a threshold value greater than a value of image data corresponding to a highest frequency and a maximum value of image data.

5. The organ imaging device of claim 4, wherein
the threshold value equals 1.1 to 1.3 times the value of image data corresponding to the highest frequency.

6. The organ imaging device of any one of claims 1 to 5, wherein
the detector extracts a contour line of the organ from the taken image of the organ, and then sets a detection area for detection of the degree of gloss with reference to the extracted contour line, and
the image data of the blue component is extracted from pixels in the detection area.
